# EUROPEAN PATENT APPLICATION

(11) **EP 4 111 947 A1**
(43) Date of publication of application: **04.01.2023**
(21) Application number: 21760584.9
(22) Date of filing: 19.01.2021
(51) Int. Cl.: A61B 5/00, A61B 5/1455, A61B 5/1491, G01N 21/17

(54) **BIOLOGICAL INFORMATION ACQUISITION DEVICE, BIOLOGICAL INFORMATION ACQUISITION SYSTEM, AND BIOLOGICAL INFORMATION ACQUISITION METHOD**

(30) Priority: 28.02.2020 JP 2020033402
(71) Applicant: Sony Group Corporation, Tokyo 108-0075 (JP)
(72) Inventor: ITO Atsushi, Tokyo 108-0075 (JP)
(74) Representative: D Young & Co LLP
(86) International application number: PCT/JP2021/001557
(87) International publication number: WO 2021/171831

(57) **Abstract**

To provide a technology that is noninvasive but can acquire information with high accuracy with a simple configuration.

Provided is a biological information acquisition device including a measurement unit that measures a radiation spectrum emitted from a measurement part, and an information processing unit that acquires biological information on the basis of a plurality of measurement results obtained from the measurement unit. Furthermore, also provided is a biological information acquisition system including a measurement apparatus that measures a radiation spectrum emitted from a measurement part, and an information processing apparatus that acquires biological information on the basis of a plurality of measurement results obtained from the measurement apparatus. Moreover, also provided is a biological information acquisition method including a measurement step of measuring a radiation spectrum emitted from a measurement part, and an information processing step of acquiring biological information on the basis of a plurality of measurement results obtained in the measurement step.

## Description

### TECHNICAL FIELD

The present technology relates to a biological information acquisition device, a biological information acquisition system, and a biological information acquisition method.

### BACKGROUND ART

If various pieces of component information can be noninvasively measured, various effects can be expected. For example, a diabetic patient needs to take a blood sample by performing needling on a finger several times a day, and this involves pain and has a problem that the skin of the needled part becomes thicker. Further, in the field of professional sports, the lactic acid level is measured likewise with an invasive tool in order to avoid overtraining, and also this is performed by taking a blood sample and is therefore not used very often by enthusiasts of amateur sports. Further, if the cholesterol level in blood has risen, a critical disorder such as cerebral infarction may be caused; however, in the conventional technology, it is difficult to noninvasively measure cholesterol with good accuracy.

Hence, if there is a means capable of measuring these components simply, health care can be easily performed, and the occurrence of diseases involving high medical expenses can be prevented. Thus, it is strongly hoped that a means for noninvasively measuring various pieces of component information will appear, and in response to this, various technologies have been proposed. Among those relatively considerably studied, there are a technology using a near-infrared spectrum, ultrasonic waves, or radio waves and a technology in which these are combined; however, all these means have low selectivity to in-body substances, and are not of practical use. On the other hand, a method using a mid-infrared spectrum has relatively high selectivity, but this is expensive because of the use of a laser and furthermore has biological safety concerns due to laser exposure.

Thus, for example, Patent Document 1 discloses a technology in which heat radiation is used to noninvasively measure in-body components. However, in a case where heat radiation is used, it is necessary to accurately monitor and manage the temperature of a human being or an apparatus. Hence, for example, Patent Document 2 discloses an apparatus in which a window member is provided with a function of temperature control. Further, for example, Patent Document 3 discloses an apparatus in which temperature sensors are installed in various places.

Further, there is known a problem that the concentrations of substances such as blood sugar and cholesterol described above are lower than those of cells and the like included in a human being and cannot ensure good S/N ratios. Thus, for example, Patent Document 4 discloses a technology in which the S/N ratio is improved by raising and lowering the surface temperature of the skin.

### CITATION LIST

### PATENT DOCUMENT

Patent Document 1: U.S. Patent No. 5,615,672
Patent Document 2: U.S. Patent No. 6,633,771
Patent Document 3: U.S. Patent No. 8,611,975
Patent Document 4: U.S. Patent No. 6,072,180

### SUMMARY OF THE INVENTION

### PROBLEMS TO BE SOLVED BY THE INVENTION

Although thus far technologies for noninvasively measuring various pieces of component information have been proposed, the apparatus is expensive, and further all of them have needed large-scale apparatuses to make measurement with good accuracy.

Thus, a main object of the present technology is to provide a technology that is noninvasive but can acquire information with high accuracy with a simple configuration.

### SOLUTIONS TO PROBLEMS

That is, the present technology provides a biological information acquisition device including a measurement unit that measures a radiation spectrum emitted from a measurement part, and an information processing unit that acquires biological information on the basis of a plurality of measurement results obtained from the measurement unit.

In the present technology, there may be a plurality of measurement parts. In this case, the measurement unit may be provided for each measurement part. In this case, the measurement unit may include a thermal conductor.

In this case, the measurement unit may include an optical filter. In this case, the measurement unit may include a light reception element that receives light transmitted through the optical filter. Furthermore, in this case, the measurement unit may include a lens that makes light incident on the optical filter substantially parallel.

Furthermore, in this case, the biological information acquisition device according to the present technology, may further include a spectroscope that separates a radiation spectrum incident on the measurement unit into spectral components.

Moreover, in this case, the biological information acquisition device according to the present technology, may further include a temperature control unit that controls temperature of the measurement unit provided for each measurement unit. In this case, a Peltier effect may be used for control of the temperature. In this case, the Peltier element may be placed such that heat transfer occurs in a direction substantially parallel to a plane including the plurality of measurement parts. Furthermore, in this case, the temperature control unit may change temperature over time. In this case, the temperature control unit may raise and lower temperature over time. In this case, the temperature control units may be adjusted to different temperatures. In this case, control of the temperature may be based on adjustment of a current flowing through the temperature control unit.

In addition, in this case, a current adjustment unit that adjusts a current flowing through the temperature control unit may be provided for each of the temperature control units.

Furthermore, in the present technology, the information processing unit acquires biological information by using differential of the plurality of measurement results.

Furthermore, the present technology also provides a biological information acquisition system including a measurement apparatus that measures a radiation spectrum emitted from a measurement part, and an information processing apparatus that acquires biological information on the basis of a plurality of measurement results obtained from the measurement apparatus.

Moreover, the present technology also provides a biological information acquisition method including a measurement step of measuring a radiation spectrum emitted from a measurement part, and an information processing step of acquiring biological information on the basis of a plurality of measurement results obtained in the measurement step.

### BRIEF DESCRIPTION OF DRAWINGS

Fig. 1 is a diagram showing a configuration example of an information acquisition apparatus.
Fig. 2 is a cross-sectional view showing a configuration example of a measurement unit.
Fig. 3 is a cross-sectional view showing modification example 1 of the configuration of the measurement unit.
Fig. 4 is a cross-sectional view showing modification example 2 of the configuration of the measurement unit.
Fig. 5 is a graph showing an example of temperature change in the vicinity of measurement units in a case where a constant current of 1.5 A is certificated to temperature control units employing a Peltier structure shown in Fig. 1.
Fig. 6 is a graph showing an example of a driving method for each of the measurement units installed in the configuration shown in Fig. 1.
Fig. 7 is a diagram showing a modification example of the configuration of the temperature control unit.
Fig. 8 is a diagram showing a configuration example of an information processing unit.
Fig. 9 is a diagram showing a configuration example of an information acquisition system.
Fig. 10 is a flowchart showing an example of an information acquisition method.

### MODE FOR CARRYING OUT THE INVENTION

Hereinbelow, preferred forms for implementing the present technology are described.

Note that the embodiments described below show representative embodiments of the present technology, and the scope of the present technology should not be construed narrowly because of these. The description of the present technology is given in the following order.
1. Information acquisition device 10
   1-1. Configuration of information acquisition device 10
   1-2. Measurement unit 11
   1-3. Modification example 1 of configuration of measurement unit 11
   1-4. Modification example 2 of configuration of measurement unit 11
   1-5. Temperature control unit 12
   1-6. Modification example of temperature control unit 12
   1-7. Information processing unit 14
2. Information acquisition system 20
3. Information acquisition method

### 1. Information acquisition device 10

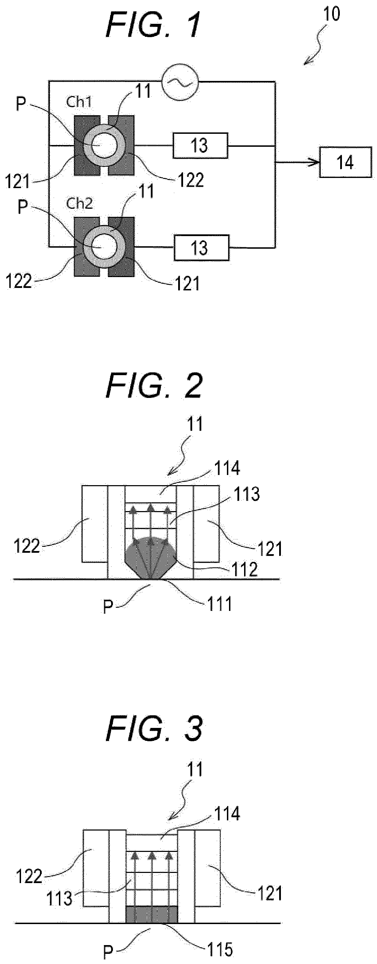

Fig. 1 is a schematic diagram showing a configuration example of an information acquisition device 10.

Hereinbelow, the configuration of the information acquisition device 10 according to the present embodiment is described. Note that the embodiment shows a preferred example of the present technology, and the information acquisition device 10 according to the present technology is not limited to this configuration. Further, in the embodiments shown below, a measurement part P is not included in the embodiments.

### 1-1. Configuration of information acquisition device 10

The information acquisition device 10 according to the present embodiment includes measurement units 11 each of which measures a radiation spectrum emitted from a measurement part P and an information processing unit 14 that acquires information on the basis of a plurality of measurement results obtained from the measurement units 11.

In the present specification, "information" refers to all kinds of information obtained from the measurement unit 11. In the present technology, "information" is, among these, particularly preferably information regarding organic substances; the information is, among the pieces of information regarding organic substances, particularly preferably biological information, food component information, and component information of plastics, paints, or the like, and more preferably biological information. The biological information is preferably in-vivo information of an animal including a human being. Examples of the in-vivo information include glucose (dextrose) concentration, cholesterol concentration, triglyceride concentration, lactic acid concentration, creatinine concentration, urea concentration, uric acid concentration, peptide concentration, oxygen partial pressure, carbon dioxide partial pressure, hydroxide ion concentration, medicine concentration, and the like.

In the present technology, as shown in Fig. 1, the number of measurement parts P is preferably plural (two or more). By using such a configuration, a plurality of measurement results can be obtained at the same time, and measurement accuracy is improved.

Hereinbelow, each part of the information acquisition device 10 according to the present embodiment is described in detail.

### 1-2. Measurement unit 11

The measurement unit 11 is a part that measures a radiation spectrum emitted from the measurement part P. In the present technology, the "measurement part" is not particularly limited; however, in a case where information that can be acquired in the measurement unit 11 is biological information, the outer layer of the skin, a body fluid, part of a tissue, or the like of an animal including a human being is given.

In the present embodiment, the measurement unit 11 is provided for each measurement part P. Thus, radiation spectra can be measured at the same time in a plurality of measurement parts P, and measurement accuracy is improved.

Fig. 2 is a cross-sectional view showing a configuration example of the measurement unit 11. In the present embodiment, the measurement unit 11 is a tubular thermal conductor. By using a tubular shape, the measurement part can be surrounded, and the entrance of stray light from the outside can be prevented and the temperature distribution of the entire measurement part can be made uniform; therefore, high accuracy measurement can be achieved.

Note that although in the present embodiment the measurement unit 11 is kept in contact with the measurement part P, the present technology is not limited thereto, and the distance from the measurement part P is not limited as long as the temperature of the measurement part P can be appropriately managed. Thus, in the present technology, another member or the like may be inserted between the measurement unit 11 and the measurement part P; examples of the another member or the like include a coating member or the like for suppressing metal allergy.

In the present technology, the thermal conductor included in the measurement unit 11 is preferably a metal material. Examples of the material of the metal material include aluminum, copper, gold, silver, a graphite sheet, and the like. From the viewpoint of enhancing the thermal conductivity even more effectively, the material of the metal material is preferably aluminum, copper, or gold, and is more preferably copper.

In the present embodiment, one end of the measurement unit 11 in a tubular shape is used while being kept in contact with the measurement part P, and a temperature control unit 12 described later is used in combination; thus, the surface temperature of the measurement part P can be controlled to a desired temperature. In the present embodiment, a pinhole 111 is formed in an opening on the side in contact with the measurement part P of the measurement unit 11, and furthermore a lens 112 is placed such that the focus is placed on the pinhole 111. The lens 112 is not particularly limited as long as the light incident on an optical filter 113 described later can be made substantially parallel.

The material contained in the lens 112 is preferably a material transparent to light emission wavelengths of a human being (around approximately 10 µm), and examples include Si (silicon), Ge (germanium), ZnSe (zinc selenide), and the like. Further, in cases such as where the material of the lens has skin sensitizing properties to the body of an animal including a human being, for example, surface treatment may be performed on the lens 112, as appropriate.

The light that is incident on the lens 112 and has become substantially parallel is incident on an optical filter 113. By providing the optical filter 113, light having a desired optical characteristic (for example, a wavelength or the like) can be separated with high accuracy.

The optical filter 113 preferably has wavelength selectivity. For the wavelength selectivity, the wavelength may be selected in accordance with the wavelength of light that should not be transmitted by the optical filter 113, as appropriate.

Examples of the optical filter having wavelength selectivity include a wavelength tunable filter including a wavelength sweep type, a wavelength fixed filter, and the like. Examples of the wavelength tunable filter include a resonator waveguide filter and a MEMS Fabry-Perot filter; among these, examples of the wavelength sweep filter include a MEMS Fabry-Perot filter.

In the present embodiment, a wavelength sweep filter is preferably selected as the optical filter 113. Here, a living body, food, or the like contains a plurality of substances, and is therefore generally observed in a form in which a plurality of radiation spectra is superimposed. Hence, in the information processing unit 14 described later, the concentration of each substance is estimated by signal processing that performs the separation of variables using a radiation spectrum of each substance as the base; however, the wavelength sweep filter has a larger amount of information than the wavelength fixed filter, and enables concentration estimation with higher accuracy.

In the present embodiment, the measurement unit 11 includes a light reception element 114 that receives light transmitted through the optical filter 113. By the light reception element 114 being housed in the measurement unit 11, for example, the temperatures of measurement parts of the skin or the like can be equalized, and a temperature distribution between measurement units 11 can be eliminated and unnecessary heat radiation can be suppressed.

The light reception element 114 is not particularly limited as long as it has sensitivity to the range of the optical characteristic intended to be measured (for example, a wavelength range or the like). Specific examples include a thermopile, a pyroelectric sensor, an MCT sensor, and the like.

The optical characteristic to be extracted by the optical filter 113 may be selected on the basis of information intended to be acquired by the measurement unit 11, as appropriate. For example, in a case where it is intended to measure glucose concentration among pieces of in-vivo information, the optical characteristic to be extracted may be, for example, a wavelength of 9.23 µm. Further, in a case where it is intended to measure cholesterol concentration, the optical characteristic to be extracted may be, for example, a wavelength of 5.7 µm.

Further, the optical characteristic to be extracted may be, for example, a wavelength serving as a standard of in-vivo information intended to be measured. Examples of the wavelength include 5.1 µm, which is the light emission wavelength of water, and the like.

### 1-3. Modification example 1 of configuration of measurement unit 11

Fig. 3 is a cross-sectional view showing modification example 1 of the configuration of the measurement unit 11.

In the present embodiment, only the configuration of the measurement unit 11 is different, and the other configurations are similar to those of the embodiment shown in Fig. 1.

In the present embodiment, not a pinhole but a measurement window 115 is formed in an opening portion on the side in contact with the measurement part P of the measurement unit 11 in a tubular shape. In a case where the information to be acquired by the measurement unit 11 is in-vivo information, particularly a radiation spectrum emitted from the body surface of an animal including a human being is assumed to be diffused light; therefore, an optical filter 113 utilizing a light interference phenomenon is difficult to install on the front side of a photodetector as shown in Fig. 2. Thus, in the present embodiment, an absorbing filter that absorbs an optical characteristic other than a desired one (for example, a wavelength or the like) may be installed as the optical filter 113. By using such a configuration, the area in contact with the measurement part P can be expanded and the amount of light incident on the photodetector is increased, and measurement accuracy can be improved.

In the present embodiment, the measurement window 115 does not particularly need to be in a lens shape, but may be simply a parallel flat plate; thus, in the present embodiment, the measurement unit 11 can be manufactured at low cost.

### 1-4. Modification example 2 of configuration of measurement unit 11

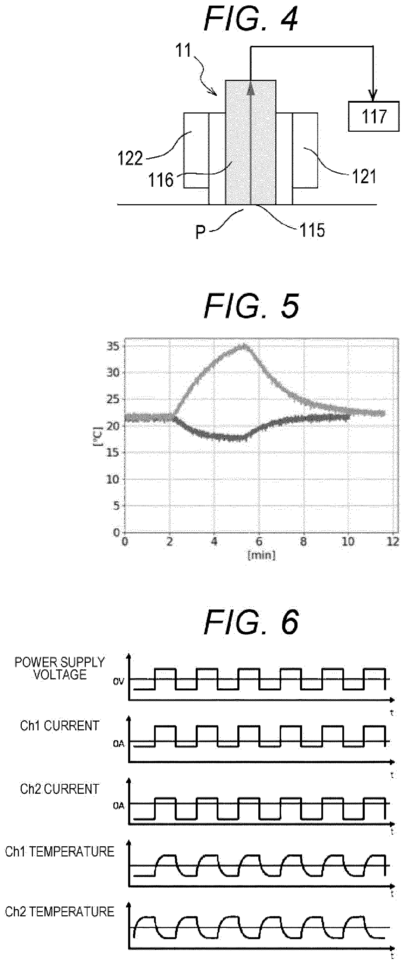

Fig. 4 is a cross-sectional view showing modification example 2 of the configuration of the measurement unit 11.

In the present embodiment, only the configuration of the measurement unit 11 is different, and the other configurations are similar to those of the embodiment shown in Fig. 1.

In the present embodiment, a radiation spectrum emitted from the measurement part P is introduced into a spectroscope 117 via an optical fiber 116. Measurement with very high accuracy can be performed by thus connecting the output end of the optical fiber 116 to the spectroscope 117. Examples of the spectroscope 117 include an FT-IR spectroscope, a dispersive spectroscope, and the like.

### 1-5. Temperature control unit 12

The present embodiment shown in Fig. 1 includes temperature control units 12 each of which grasps the tubular measurement unit 11 and controls the temperature of the measurement unit 11. The temperature control unit 12 includes an n-type semiconductor 121 and a p-type semiconductor 122.

In the present embodiment, the temperature control unit 12 preferably uses the Peltier effect to control the temperature of the measurement unit 11. The "Peltier effect" is generally known as a phenomenon in which when different conductors with high conductivity are joined and a current is passed from one conductor toward another conductor, heat absorption occurs in the joint portion.

Examples of the method that uses the Peltier effect to control the temperature of the measurement unit 11 include the adjustment of a current flowing through the temperature control unit 12. More specifically, in a case where one semiconductor is the cooling side, the other semiconductor is the heat generation side; for example, when a current is passed from the n-type semiconductor 121 toward the p-type semiconductor 122, the measurement unit 11 is cooled; conversely, when a current is passed from the p-type semiconductor 122 toward the n-type semiconductor 121, the measurement unit 11 is heated. In the present embodiment, the temperature control of the measurement unit 11 using the Peltier effect can be achieved by thus fabricating the temperature control unit 12 by using the n-type semiconductor 121 and the p-type semiconductor 122.

The n-type semiconductor 121 and the p-type semiconductor 122 may each be, for example, a compound in which Sb (antimony), In (indium), Se (selenium), or the like is added to a bismuth tellurium (Bi2Te3)-based compound.

Fig. 5 is a graph showing an example of temperature change in the vicinity of measurement units 11 in a case where a constant current of 1.5 A is certificated to temperature control units 12 employing a Peltier structure shown in Fig. 1. As shown in Fig. 5, it can be seen that the temperature in the vicinity of the measurement unit 11 rises or drops depending on the direction of current.

However, although the same current is passed, the amount of temperature change is larger at the time of rising. This is because Joule heat is generated simultaneously with the Peltier effect. Thus, if an equal current is passed at the time of temperature rising and at the time of temperature lowering, the temperature rises too high at the time of temperature rising. Thus, in order to suppress the rising of temperature at the time of temperature rising, a current adjustment unit 13 is preferably provided for each temperature control unit 12.

In the present embodiment, the current adjustment unit 13 may be, for example, a constant current circuit 123. Thus, the amount of current flowing through the temperature control unit 12 can be controlled to a desired amount, and an event where the temperature rises too high at the time of temperature rising can be prevented.

Fig. 6 is a graph showing an example of a driving method for each of the measurement units 11 (hereinafter, referred to as "Ch1" and "Ch2", respectively) installed in the configuration shown in Fig. 1. For the power source, for example, a case where positive and negative voltages (for example, 5 V) around 0 V are rectangularly driven in an alternately manner is conceivable. At this time, the current of each of one measurement unit 11 (Ch1) and the other measurement unit 11 (Ch2) is restricted by the constant current circuit 123 in synchronization with the power source so that the amount of current at the time of heating is kept low.

Further, the installation directions of the n-type semiconductors 121 and the p-type semiconductors 122 of the measurement unit 11 (Ch1) and the measurement unit 11 (Ch2) are set to opposite directions as shown in Fig. 1; thus, as shown in Fig. 5, temperature change can be produced while the measurement unit 11 (Ch1) and the measurement unit 11 (Ch2) are set in antiphase. By using such a configuration, while a single power source is used, the temperature can be controlled to appropriate temperatures for each measurement unit 11, and the downsizing and cost reduction of the entire apparatus can be achieved.

In the present embodiment, the temperature control unit 12 preferably changes temperature over time. Thus, the absorption or release of a radiation spectrum in the measurement part P is stimulated, and information can be acquired with good accuracy. Specifically, for example, the temperature control unit 12 preferably raises and lowers temperature over time. In a case where the biological information is, for example, glucose concentration, if the measurement part P is cooled, the absorption of a radiation spectrum is stimulated, and if the measurement part P is heated, the release of a radiation spectrum is stimulated.

Further, in the present embodiment, it is preferable that the temperature control units 12 provided for the measurement units 11 be adjusted to different temperatures. Thus, heat can be alternately transferred between a plurality of temperature control units 11, and the temperature of the body surface or the like can be efficiently raised and lowered. For example, in a case where the measurement part P is the body surface, if the body surface temperature is lowered, heat radiation from an in-body deep portion is observed, and if the body surface temperature is raised, a large amount of radiation from the interior of the body can be observed; therefore, in the information processing unit 14 described later, the amount of absorption of the body surface tissue can be found with good accuracy by using differential signals from between a plurality of measurement units 11.

In this case, by performing heat transfer between temperature control units 12 with a constant period, the information processing unit 14 described later can take out only signals synchronized with the heat transfers, and the S/N ratio can be improved in the time axis direction.

Further, in the present embodiment, in a case where the information is in-vivo information of a human being, the temperature in the high temperature state of the measurement unit 11 controlled by the respective temperature control unit 12 is preferably set such that the body temperature of a deep portion of the human being is above approximately 37 degrees. This is because thus the radiation spectrum obtained in the light reception element 114 can be expected to be only a radiation spectrum generated from the body surface and, by comparison with the measurement unit 11 in the cooling state, can be determined to be a signal derived from the interior of the body.

### 1-6. Modification example of temperature control unit 12

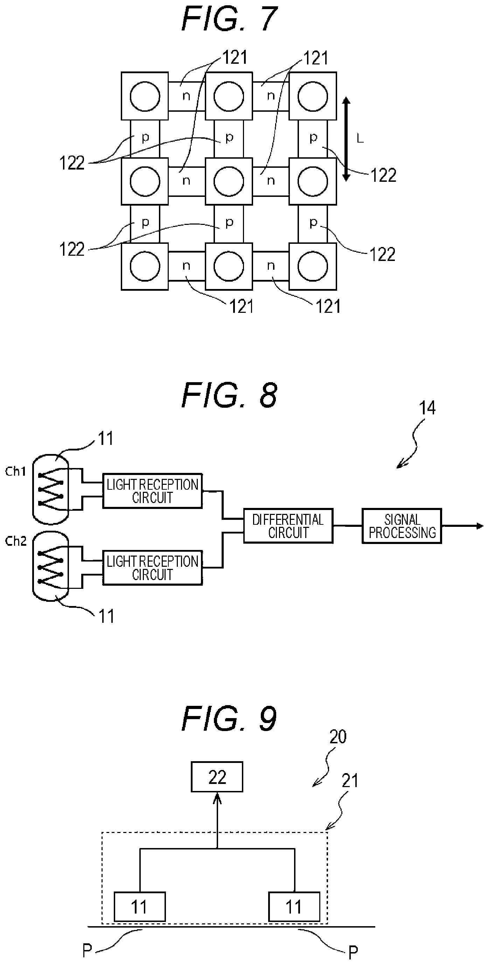

Fig. 7 is a diagram showing a modification example of the configuration of the temperature control unit 12.

In the present embodiment, only the configuration of the temperature control unit 12 is different, and the other configurations are similar to those of the embodiment shown in Fig. 1.

In the present embodiment, it is preferable that the temperature control units 12 be arranged such that heat transfer occurs in a direction substantially parallel to a plane including the plurality of measurement parts. Specifically, for example, as shown in Fig. 7, n-type semiconductors 121 or p-type semiconductors 122 are arranged facing each other, on the other hand the p-type semiconductor 122 is placed on a side facing the n-type semiconductor 121 at substantially 90 degrees, and the n-type semiconductor 121 and the p-type semiconductor 122 are alternately joined and arranged facing each other at substantially 90 degrees via the respective measurement unit 11. In this case, each measurement unit 11 can function as both a thermal conductor and an electrode.

By using an arrangement like that shown in Fig. 7, the structure becomes what is called a n-type Peltier structure; thus, heat transfer can be produced in a direction substantially parallel to a plane including a plurality of measurement parts P, and it is unnecessary to perform heat discharge for cooling. Therefore, there is no need to perform heat radiation to the outside of the system, and the downsizing of the apparatus can be achieved.

Further, particularly in a case where the measurement part P is the body surface of a human being, the interval (pitch) L between measurement units 11 (see L of Fig. 7) is preferably not more than the cold spot interval of the skin. This is in order not to give discomfort to the human being when heating or cooling the body surface of the human being. In particular, if the body surface of the human being is cooled to 15°C or less, the human being feels a sense of pain. Thus, it is preferable to reduce the physical distance between measurement units 11.

The interval L is preferably 2 mm or less, and more preferably 0.5 mm or less. By setting the interval L to 2 mm or less, a cold spot and a warm spot, which are human sensory organs, can be simultaneously stimulated, and the giving of a sense of pain to the human being can be avoided even if the body surface of the human being is cooled to approximately 15°C.

### 1-7. Information processing unit 14

The information processing unit 14 is a part that acquires biological information on the basis of a plurality of measurement results obtained from the measurement units 11. In the present embodiment, a plurality of measurement results is preferably measurement results obtained from the plurality of measurement units 11.

Fig. 8 is a diagram showing a configuration example of an information processing unit 14. In the present embodiment, the output of the light reception element 114 provided for each measurement unit 11 is made into an electrical signal by a light reception circuit, and is amplified. After that, differential amplification between measurement units 11 is performed, and then connection is made to a signal processing circuit. The signal processing circuit calculates information. By thus using differential between measurement units 11, electrical common-mode noise occurring in the information acquisition device 10 can be removed. Further, particularly in a case where the information is biological information, also a disturbance factor such as heartbeats/respiration peculiar to a living body can be excluded.

Here, the above-described Patent Document 4 (U.S. Patent 08/816,723) discloses a technique in which in-body components are analyzed with high accuracy by reducing the body surface temperature to less than the body temperature of a deep portion; this creates a high temperature state and a low temperature state by using one measurement unit, and therefore the time at which comparison is performed is different.

However, particularly a human being has motion of respiration and pulsation of blood, and therefore it is desirable that the comparison between the high temperature state and the low temperature state be performed at almost the same time. Hence, in the present embodiment, differential of measurement results obtained from a plurality of (at least two or more) measurement units 11 is performed, and thus comparison almost at the same time can be performed.

However, the measurement parts P of the measurement units 11 are spatially different, and therefore the same measurement result is not necessarily obtained from the plurality of measurement units 11. Hence, it is preferable that a characteristic of a radiation spectrum in each measurement part P be acquired by driving each measurement unit 11 in a temperature profile like that shown in Fig. 6 and correction be performed. For example, if the light emission intensity at the measurement position in a measurement unit 11 (Ch2) is smaller than the intensity in a measurement unit 11 (Ch1), processing such as applying a gain to the component of the measurement unit 11 (Ch2) in advance may be performed when performing differential.

### 2. Information acquisition system 20

Fig. 9 is a diagram showing a configuration example of an information acquisition system 20.

Hereinbelow, the configuration of the information acquisition system 20 according to the present embodiment is described. Note that the embodiment shows a preferred example, and the information acquisition system 20 according to the present technology is not limited to this configuration.

In the present embodiment, the information acquisition system 20 includes a measurement apparatus 21 that measures a radiation spectrum emitted from the measurement part P. The measurement apparatus 21 may include, for example, a plurality of measurement units 11 described above. The measurement unit 11 is similar to that described above, and therefore a description is omitted herein.

Further, in the present embodiment, the information acquisition system 20 includes an information processing apparatus 22 that acquires information on the basis of a plurality of measurement results obtained from the measurement apparatus 21. The processing performed in the information processing apparatus 22 may be, for example, similar to the processing performed in the information processing unit 14 described above. The information processing unit 14 is similar to that described above, and therefore a description is omitted herein.

### 3. Information acquisition method

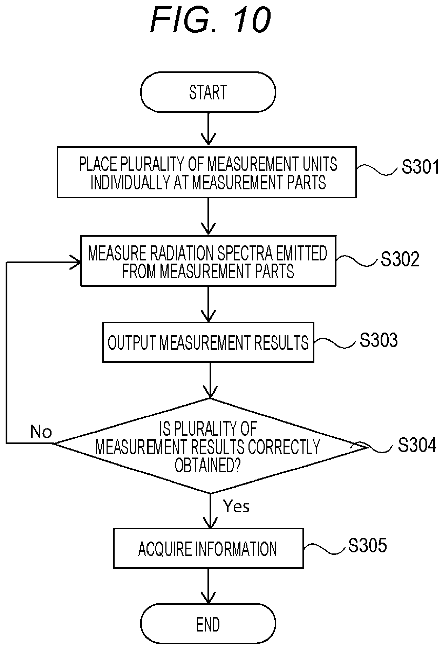

Fig. 10 is a flowchart showing an example of an information acquisition method.

Hereinbelow, the configuration of the information acquisition method according to the present embodiment is described. Note that the embodiment shows a preferred example, and the information acquisition method according to the present technology is not limited to this configuration.

In the present embodiment, first, a plurality of measurement units 11 is placed individually at measurement parts P (S301). Next, radiation spectra emitted from the measurement units 11 are simultaneously measured (S302). Next, the measurement results are outputted to the information processing unit 14 (S303). The information processing unit 14 checks whether a plurality of measurement results has been correctly acquired or not; in a case where it has not been acquired, the procedure returns to S302 (S304). In a case where it has been correctly acquired, for example, differential of a plurality of measurement results is performed, and the information of interest (for example, in-vivo information such as glucose concentration, etc.) is acquired (S305).

Additionally, the present technology may be configured as below.
[1] An information acquisition device including:
   a measurement unit that measures a radiation spectrum emitted from a measurement part; and
   an information processing unit that acquires information on the basis of a plurality of measurement results obtained from the measurement unit.
[2] The information acquisition device according to [1], in which there is a plurality of measurement parts.
[3] The information acquisition device according to [2], in which the measurement unit is provided for each measurement part.
[4] The information acquisition device according to [3], in which the measurement unit includes a thermal conductor.
[5] The information acquisition device according to [4], in which the measurement unit includes an optical filter.
[6] The information acquisition device according to [5], in which the measurement unit includes a light reception element that receives light transmitted through the optical filter.
[7] The information acquisition device according to [5], in which the measurement unit includes a lens that makes light incident on the optical filter substantially parallel.
[8] The information acquisition device according to [4], further including: a spectroscope that separates a radiation spectrum incident on the measurement unit into spectral components.
[9] The information acquisition device according to any one of [4] to [8], in which a temperature control unit that controls temperature of the measurement unit is provided for each measurement unit.
[10] The information acquisition device according to [9], in which a Peltier effect is used for control of the temperature.
[11] The information acquisition device according to [10], in which the temperature control unit is placed such that heat transfer occurs in a direction substantially parallel to a plane including the plurality of measurement parts.
[12] The information acquisition device according to [10] or [11], in which the temperature control unit changes temperature over time.
[13] The information acquisition device according to [12], in which the temperature control unit raises and lowers temperature over time.
[14] The information acquisition device according to [13], in which the temperature control units are adjusted to different temperatures.
[15] The information acquisition device according to any one of [9] to [14], in which adjustment of the temperature is based on adjustment of a current flowing through the temperature control unit.
[16] The information acquisition device according to [15], in which a current adjustment unit that adjusts a current flowing through the temperature control unit is provided for each of the temperature control units.
[17] The information acquisition device according to any one of [1] to [16], in which the information processing unit acquires information by using differential of the plurality of measurement results.
[18] An information acquisition system including:
   a measurement apparatus that measures a radiation spectrum emitted from a measurement part; and
   an information processing apparatus that acquires information on the basis of a plurality of measurement results obtained from the measurement apparatus.
[19] An information acquisition method including:
   a measurement step of measuring a radiation spectrum emitted from a measurement part; and
   an information processing step of acquiring information on the basis of a plurality of measurement results obtained in the measurement step.

Additionally, the present technology may also be configured as below.
[1] A biological information acquisition device including:
   a measurement unit that measures a radiation spectrum emitted from a measurement part; and
   an information processing unit that acquires biological information on the basis of a plurality of measurement results obtained from the measurement unit.
[2] The biological information acquisition device according to [1], in which there is a plurality of measurement parts.
[3] The biological information acquisition device according to [2], in which the measurement unit is provided for each measurement part.
[4] The biological information acquisition device according to [3], in which the measurement unit includes a thermal conductor.
[5] The biological information acquisition device according to [4], in which the measurement unit includes an optical filter.
[6] The biological information acquisition device according to [5], in which the measurement unit includes a light reception element that receives light transmitted through the optical filter.
[7] The biological information acquisition device according to [5], in which the measurement unit includes a lens that makes light incident on the optical filter substantially parallel.
[8] The biological information acquisition device according to [4], further including: a spectroscope that separates a radiation spectrum incident on the measurement unit into spectral components.
[9] The biological information acquisition device according to any one of [4] to [8], in which a temperature control unit that controls temperature of the measurement unit is provided for each measurement unit.
[10] The biological information acquisition device according to [9], in which a Peltier effect is used for control of the temperature.
[11] The biological information acquisition device according to [10], in which the temperature control unit is placed such that heat transfer occurs in a direction substantially parallel to a plane including the plurality of measurement parts.
[12] The biological information acquisition device according to [10] or [11], in which the temperature control unit changes temperature over time.
[13] The biological information acquisition device according to [12], in which the temperature control unit raises and lowers temperature over time.
[14] The biological information acquisition device according to [13], in which the temperature control units are adjusted to different temperatures.
[15] The biological information acquisition device according to any one of [9] to [14], in which adjustment of the temperature is based on adjustment of a current flowing through the temperature control unit.
[16] The biological information acquisition device according to [15], in which a current adjustment unit that adjusts a current flowing through the temperature control unit is provided for each of the temperature control units.
[17] The biological information acquisition device according to any one of [1] to [16], in which the information processing unit acquires information by using differential of the plurality of measurement results.
[18] A biological information acquisition system including:
   a measurement apparatus that measures a radiation spectrum emitted from a measurement part; and
   an information processing apparatus that acquires biological information on the basis of a plurality of measurement results obtained from the measurement apparatus.
[19] A biological information acquisition method including:
   a measurement step of measuring a radiation spectrum emitted from a measurement part; and
   an information processing step of acquiring information on the basis of a plurality of measurement results obtained in the measurement step.

### REFERENCE SIGNS LIST

- 10: Information acquisition device
- 11: Measurement unit
- 111: Pinhole
- 112: Lens
- 113: Optical filter
- 114: Light reception element
- 115: Measurement window
- 116: Optical fiber
- 117: Spectroscope
- 12: Temperature control unit
- 121: n-type semiconductor
- 122: p-type semiconductor
- 123: Constant current circuit
- 13: Current adjustment unit
- 14: Information processing unit
- 20: Information acquisition system
- 21: Measurement apparatus
- 22: Information processing apparatus
- P: Measurement part
- L: Interval (pitch) between measurement units

## Claims

1. A biological information acquisition device comprising:
a measurement unit that measures a radiation spectrum emitted from a measurement part; and
an information processing unit that acquires biological information on a basis of a plurality of measurement results obtained from the measurement unit.

2. The biological information acquisition device according to claim 1, wherein there is a plurality of measurement parts.

3. The biological information acquisition device according to claim 2, wherein the measurement unit is provided for each measurement part.

4. The biological information acquisition device according to claim 3, wherein the measurement unit includes a thermal conductor.

5. The biological information acquisition device according to claim 4, wherein the measurement unit includes an optical filter.

6. The biological information acquisition device according to claim 5, wherein the measurement unit includes a light reception element that receives light transmitted through the optical filter.

7. The biological information acquisition device according to claim 5, wherein the measurement unit includes a lens that makes light incident on the optical filter substantially parallel.

8. The biological information acquisition device according to claim 4, further comprising: a spectroscope that separates a radiation spectrum incident on the measurement unit into spectral components.

9. The biological information acquisition device according to claim 4, wherein a temperature control unit that controls temperature of the measurement unit is provided for each measurement unit.

10. The biological information acquisition device according to claim 9, wherein a Peltier effect is used for control of the temperature.

11. The biological information acquisition device according to claim 10, wherein the temperature control unit is placed such that heat transfer occurs in a direction substantially parallel to a plane including the plurality of measurement parts.

12. The biological information acquisition device according to claim 9, wherein the temperature control unit changes temperature over time.

13. The biological information acquisition device according to claim 12, wherein the temperature control unit raises and lowers temperature over time.

14. The biological information acquisition device according to claim 13, wherein the temperature control units are adjusted to different temperatures.

15. The biological information acquisition device according to claim 9, wherein control of the temperature is based on adjustment of a current flowing through the temperature control unit.

16. The information acquisition device according to claim 15, wherein a current adjustment unit that adjusts a current flowing through the temperature control unit is provided for each of the temperature control units.

17. The biological information acquisition device according to claim 1, wherein the information processing unit acquires biological information by using differential of the plurality of measurement results.

18. A biological information acquisition system comprising:
a measurement apparatus that measures a radiation spectrum emitted from a measurement part; and
an information processing apparatus that acquires biological information on a basis of a plurality of measurement results obtained from the measurement apparatus.

19. A biological information acquisition method comprising:
a measurement step of measuring a radiation spectrum emitted from a measurement part; and
an information processing step of acquiring biological information on a basis of a plurality of measurement results obtained in the measurement step.
